# FASCICULE DE BREVET EUROPEEN

(11) **EP 1 235 541 B1**
(45) Date de publication et mention de la délivrance du brevet: **23.07.2003**
(21) Numéro de dépôt: 00988880.1
(22) Date de dépôt: 06.12.2000
(51) Int. Cl.: A61J 1/20, A61J 1/00

(54) **PROCEDE DE FABRICATION D'UN DISPOSITIF DE CONNEXION ENTRE UN RECIPIENT ET UN CONTENANT, DISPOSITIF DE CONNEXION CORRESPONDANT ET ENSEMBLE PRET A L'EMPLOI COMPRENANT UN TEL DISPOSITIF**
VERFAHREN ZUR HERSTELLUNG EINER VERBINDUNGSVORRICHTUNG ZWISCHEN BAHÄL TER UND INHALT, ENTSPRECHENDE VERBINDUNGSVORRICHTUNG UND GEBRAUCHSFERTIGE ANORDNUNG MIT EINER SOLCHEN VORRICHTUNG
METHOD FOR PRODUCING A DEVICE FOR CONNECTING A RECEPTACLE AND A CONTAINER, CORRESPONDING CONNECTING DEVICE AND READY-FOR-USE ASSEMBLY COMPRISING A DEVICE OF THIS TYPE

(30) Priorité: 10.12.1999 FR 9915634
(43) Date de publication de la demande: 04.09.2002
(73) Titulaire: Biodome, 63500 Issoire (FR)
(72) Inventeur: ANEAS, Antoine, F-63200 Ménétrol (FR)
(74) Mandataire: Schouller, Jean-Philippe
(86) Numéro de dépôt international: FR0003413
(87) Numéro de publication internationale: WO01041699

(56) Documents cités:
- EP-A- 0 728 457
- EP-A- 0 829 250
- WO-A-97/10156
- US-A- 5 224 515

## Description

La présente invention concerne un procédé de fabrication d'un dispositif de connexion entre un récipient et un contenant, un dispositif de connexion correspondant et un ensemble prêt à l'emploi comprenant un tel dispositif.

Dans le domaine du conditionnement médical, il est connu de stocker un composant d'une préparation pharmaceutique, tel que son principe actif, dans un récipient fermé au moyen d'un bouchon réalisé en un matériau relativement mou, tel un élastomère. Un liquide est susceptible d'être introduit dans ce récipient, après perforation du bouchon, ce qui permet la dissolution ou la mise en suspension du composant contenu dans le récipient, de manière à obtenir une préparation sous forme liquide, notamment un médicament ou un vaccin, prête à être administrée à un patient.

On connaît, par exemple par WO-A-97/10 156, un dispositif de connexion entre le récipient fermé précité et un contenant. Ce dernier renferme du liquide susceptible d'être introduit dans le récipient, une fois celui-ci ouvert par perforation de son bouchon. Un tel dispositif de connexion comprend une embase adaptée pour être immobilisée sur le récipient, dans le volume intérieur de laquelle est formé un alésage interne.

Ce dispositif comprend également un piston monté coulissant dans l'alésage précité, qui est pourvu d'un organe de perforation du bouchon du récipient, telle une aiguille creuse. Ce piston est mobile entre une position de stockage, dans laquelle l'organe de perforation n'est pas en contact avec le bouchon, et une position de transfert, dans laquelle l'organe de perforation traverse ce bouchon.

Ce dispositif de connexion comporte également une coiffe comportant un couvercle à partir duquel s'étendent des parois latérales définissant une ouverture. Cette coiffe est apte à recouvrir la périphérie extérieure de l'embase, notamment durant le stockage de l'ensemble prêt à l'emploi, formé par le récipient et son dispositif de connexion.

Afin d'éviter toute migration microbienne extérieure en direction de l'organe de perforation, qui contaminerait la préparation pharmaceutique destinée à entrer en contact avec cet organe, il est connu de prévoir des moyens d'étanchéité entre les parois en regard de l'embase et de la coiffe.

A cet effet, l'enseignement de EP-A-0 728 457 prévoit de munir la paroi intérieure de la coiffe d'au moins une arête périphérique en saillie radiale vers l'intérieur. Par ailleurs, il est prévu de recouvrir la paroi extérieure en regard de l'embase au moyen d'une matière d'étanchéité de viscosité élevée. Lors du mouvement coulissant entre l'embase et la coiffe, les arêtes de cette dernière raclent la matière d'étanchéité disposée sur la coiffe et induisent la formation d'un joint circonférentiel d'étanchéité, ce qui est permis grâce à la viscosité élevée du matériau employé.

Ce procédé de fabrication connu présente cependant certains inconvénients. En effet, le joint circonférentiel ainsi formé n'assure pas une étanchéité parfaite, quelles que soient les conditions de pression relatives entre l'extérieur et l'intérieur de l'embase. En particulier, dans le cas où la pression régnant dans le volume intérieur de l'embase est supérieure à la pression atmosphérique, le joint circonférentiel précité est susceptible d'être éloigné de la zone des arêtes, de sorte que ce joint n'est alors plus à même de remplir de façon satisfaisante sa fonction d'étanchéité.

Afin de pallier ces différents inconvénients, l'invention se propose de mettre en oeuvre un procédé de fabrication d'un dispositif de connexion qui garantit d'une part une étanchéité satisfaisante entre les parois en regard de l'embase et de la coiffe et d'autre part un démontage aisé de ces deux éléments.

A cet effet, elle a pour objet un procédé de fabrication d'un dispositif de connexion entre un récipient fermé et un contenant, ce dispositif de connexion comprenant :
- une embase destinée à être immobilisée sur ce récipient,
- un piston mobile dans un alésage de ladite embase, ce piston étant pourvu d'un organe de perforation apte à traverser un bouchon du récipient, et
- une coiffe recouvrant au moins partiellement la périphérie extérieure de l'embase,
caractérisé en ce qu'on réalise un premier élément de l'ensemble formé par l'embase et la coiffe en un matériau plus dur que le second élément dudit ensemble, en ce qu'on munit le premier élément d'au moins une arête périphérique destinée à pénétrer, de façon étanche, dans une région en regard du second élément, et en ce qu'on recouvre la ou chaque arête du premier élément au moyen d'un agent de lubrification adapté pour former, une fois cette opération de recouvrement effectuée, un film de lubrification apte à rester en place sur la ou chaque arête d'étanchéité, lors du déplacement relatif de la coiffe et de l'embase, ainsi que lorsque cette coiffe et cette embase sont immobiles l'une par rapport à l'autre.

L'invention permet de réaliser les objectifs précédemment mentionnés.

La phase de recouvrement de chaque arête de l'élément dur au moyen d'un agent de lubrification conduit au mouillage de ces arêtes et, étant donné les caractéristiques de l'agent de lubrification employé, à la formation d'un film de lubrification qui se trouve immobilisé sur ces arêtes.

Faire appel à au moins une arête d'étanchéité pénétrant dans la paroi en regard de l'élément de dureté inférieure induit la formation d'une barrière mécanique, empêchant toute propagation microbienne extérieure vers le volume intérieur de l'embase. Ceci garantit une étanchéité satisfaisante au dispositif de connexion de l'invention.

La différence de dureté, sur l'échelle Shore D, entre les premier et second éléments est comprise entre 10 et 15.

Une fois la coiffe montée sur l'embase, chaque arête d'étanchéité pénètre dans la paroi en regard de l'élément le moins dur selon une profondeur d'environ 10 à 15 micromètres.

Employer un agent d'étanchéité, dont les caractéristiques d'accrochage sont telles qu'il laisse subsister un film de lubrification solidaire du ou de chaque élément qu'il recouvre, permet à un opérateur de retirer, de façon aisée, la coiffe de l'embase, immédiatement avant utilisation. Ce film présente avantageusement une épaisseur comprise entre 5 et 10 micromètres. La présence de ce film confère en outre une composante secondaire d'étanchéité, dans la mesure où ce film occupe les micro-défauts de surface de l'élément à arêtes qu'il recouvre. Par ailleurs, étant donné qu'il possède un pouvoir d'accrochage élevé, ce film ne peut être chassé en fonction des différences de pression régnant entre l'extérieur et l'intérieur de l'embase, ce qui est avantageux par rapport à l'art antérieur.

L'agent de lubrification possède avantageusement une viscosité, à 20°C, comprise entre 150 et 1 500 centistokes.

L'agent de lubrification auquel il est fait appel possède avantageusement un fort pouvoir d'étalement, grâce à sa faible tension superficielle et à sa faible viscosité. Ceci lui permet de remplir de façon satisfaisante les micro-porosités des arêtes de l'élement plus dur pénétrant dans l'élément plus mou.

L'agent de lubrification est avantageusement non miscible avec l'eau. Ce caractère hydrophobe confère un pouvoir d'étanchéité élevé au film de lubrification.

L'agent de lubrification est de préférence stable et neutre chimiquement, et peu sujet à l'évaporation. Il possède par exemple un facteur d'évaporation d'environ 0,5%, déterminé par un échantillon de 2 grammes d'agent de lubrification placé dans un récipient de 50 ml et soumis à une température de 150°C pendant 24 heures. Cet agent de lubrification possède avantageusement une tension superficielle plus forte que celle de l'air.

Cet agent de lubrification peut être une huile siliconée ou une huile fluorée.

Selon une autre caractéristique de l'invention, on recouvre le premier élément muni d'au moins une arête par pulvérisation de l'agent de lubrification.

L'invention a également pour objet un dispositif de connexion entre un récipient fermé et un contenant, ce dispositif de connexion comprenant :
- une embase destinée à être immobilisée sur ce récipient,
- un piston mobile dans un alésage de l'embase, ce piston étant pourvu d'un organe de perforation apte à traverser un bouchon du récipient, et
- une coiffe recouvrant au moins partiellement la périphérie extérieure de l'embase,
caractérisé en ce qu'un premier élement de l'ensemble formé par l'embase et la coiffe est réalisée en un matériau plus dur que le second élément dudit ensemble, en ce que ledit premier élément est muni d'au moins une arête périphérique pénétrant, de façon étanche, dans une région en regard du second élément et en ce que la ou chaque arête du premier élément est recouverte au moyen d'un film de lubrification apte à rester en place sur la ou chaque arête, lors du déplacement relatif de la coiffe et de l'embase, ainsi que lorsque cette coiffe et cette embase sont immobiles l'une par rapport à l'autre.

Selon d'autres caractéristiques de l'invention :
- la ou chaque arête du premier élément pénètre dans la paroi en regard du second élément, selon une profondeur comprise entre 10 et 15 micromètres ;
- le film de lubrification possède une épaisseur comprise entre 5 et 10 micromètres ; et
- la différence de duretés entre les premier et second éléments, sur l'échelle Shore D, est comprise entre 10 et 15.

L'invention a enfin pour objet un ensemble prêt à l'emploi comprenant un récipient fermé contenant un produit, notamment une préparation pharmaceutique, et un dispositif de connexion tel que défini ci-dessus, monté sur ledit récipient.

L'invention va être décrite ci-dessous, en référence aux dessins annexés, donnés uniquement à titre d'exemples non limitatifs et dans lesquels :
- la figure 1 est une vue en coupe longitudinale, illustrant les différents éléments constitutifs d'un dispositif de connexion conforme à l'invention ;
- la figure 2 est une vue en coupe longitudinale, illustrant une opération de pulvérisation d'un agent de lubrification appartenant au procédé de traitement de l'invention ; et
- la figure 3 est une vue en coupe longitudinale, illustrant une embase et une coiffe du dispositif de la figure 1, réalisées conformément à l'invention.

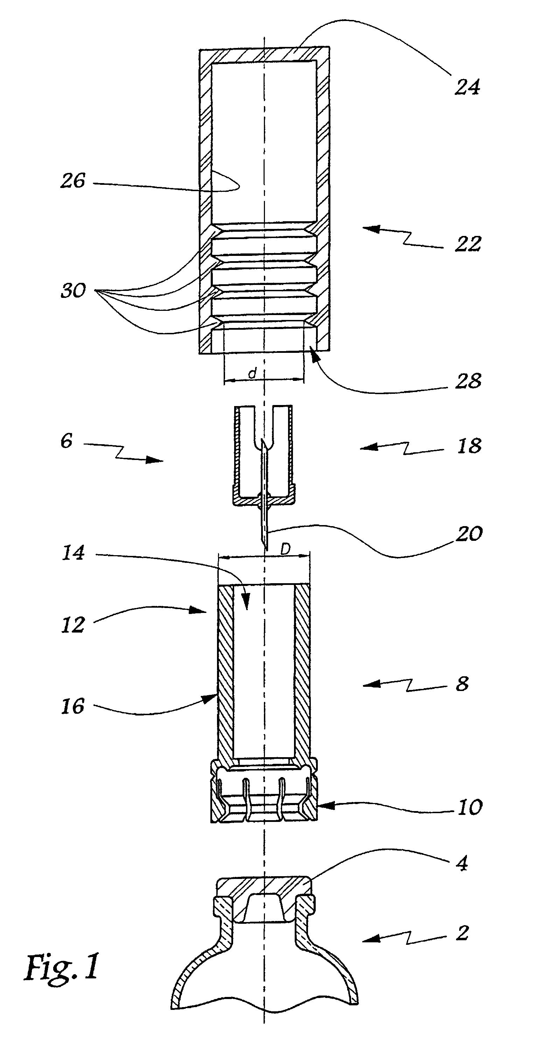

La figure 1 représente un récipient 2, par exemple un flacon en verre, contenant un produit non représenté, qui est par exemple une poudre destinée à former un vaccin buvable. Il peut également s'agir de tout autre type de préparations pharmaceutiques, en particulier de tout type de médicaments. Le récipient 2 est obturé au moyen d'un bouchon 4, réalisé de façon connue en un matériau relativement mou, tel qu'un élastomère.

La figure 1 illustre également un dispositif, désigné dans son ensemble par la référence 6, permettant la connexion entre le récipient 2 et un contenant non représenté. Ce dernier peut être une seringue renfermant un liquide destiné à mettre en solution ou en suspension le produit contenu dans le récipient 2, ou bien encore une poche souple, ou un autre flacon en verre.

Ce dispositif de connexion 6 comporte une embase 8 apte à être solidarisée, de façon connue, sur le récipient 2 par son extrémité inférieure 10. Cette dernière est prolongée par une portion principale 12 cylindrique, définissant un alésage intérieur 14 et une paroi périphérique extérieure 16, sensiblement lisse. L'embase 8 est réalisée en un matériau relativement mou, tel que du polyéthylène basse densité.

Le dispositif de connexion 6 comprend également un piston 18 apte à coulisser dans l'alésage 14 de l'embase 8. Ce piston est pourvu, de manière connue, d'une aiguille 20 de perforation du bouchon 4. Le piston 18 est mobile entre une position de stockage, dans laquelle l'aiguille n'est pas en contact avec le bouchon, et une position de transfert, dans laquelle l'aiguille 20 transperce le bouchon 4 et se trouve au contact du liquide contenu dans le récipient 2.

Le dispositif de connexion de l'invention comporte également une coiffe, désignée dans son ensemble par la référence 22, qui comporte un couvercle 24 à partir duquel s'étendent des parois latérales 26, de section transversale circulaire. Ces dernières définissent une extrémité ouverte 28 de la coiffe, destinée à l'emmanchement de l'embase 8.

La périphérie intérieure des parois latérales 26 est pourvue de plusieurs dents, ou arêtes 30, s'étendant de façon périphérique, radialement vers l'intérieur. La coiffe 22 est réalisée en un matériau plus dur que l'embase 8, par exemple du polyéthylène haute densité.

Les arêtes 30, qui sont agrandies de façon exagérée sur les figures dans un but de clarté, possèdent un diamètre intérieur d, qui est plus petit que le diamètre extérieur D de l'embase 8.

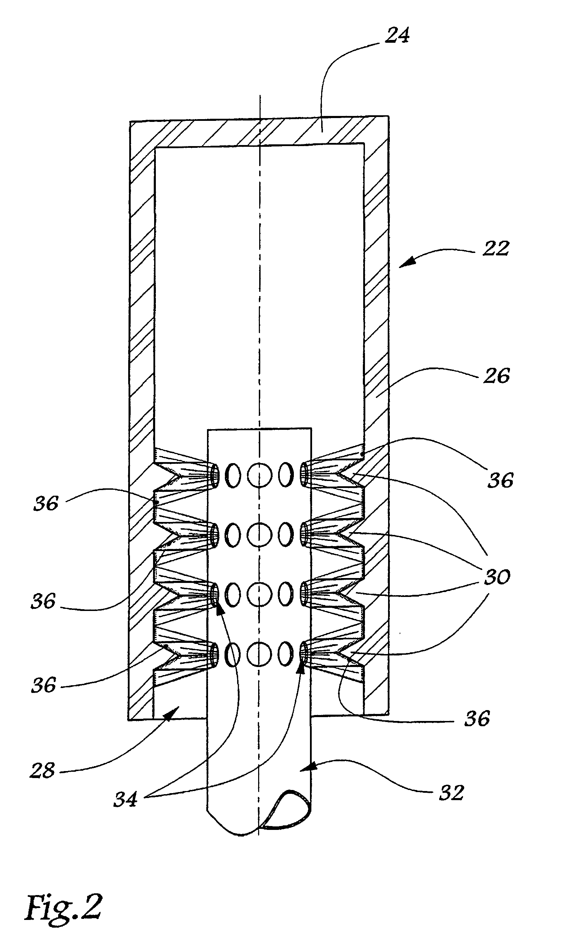

La figure 2 illustre une phase de traitement appartenant au procédé de fabrication, du dispositif de connexion 6 selon l'invention. A cet effet, on introduit, dans le volume intérieur de la coiffe 22, un dispositif de pulvérisation 32 de forme tubulaire. Ce dernier comporte plusieurs orifices radiaux 34, assurant la pulvérisation d'un agent de lubrification avec lequel est alimenté le dispositif de pulvérisation 32.

Cet agent de lubrification est par exemple une huile fluorée, telle que celle commercialisée par la société WYNN'S FRANCE sous la référence WYNNOX H4, dont la viscosité est de 180 centistokes à 20°C. On peut également faire appel à une huile siliconée, telle que celle commercialisée par la société RHONE POULENC sous la référence SILBIONE 70 047 V1000, dont la viscosité à 25°C est de 1000 centistokes et la tension superficielle de 21.2 mN/m.

La pulvérisation de cet agent de lubrification, sur la zone des arêtes 30, conduit à la formation d'un film 36 recouvrant ces arêtes 30 et leur voisinage. Etant donné que l'agent utilisé possède une bonne capacité d'accrochage sur les surfaces qu'il revêt, le film 36 possède une excellente tenue. Ainsi, ce film 36 reste solidaire des arêtes 30 qu'il recouvre, aussi bien en position de stockage, lorsque la coiffe 22 et l'embase 8 sont mutuellement immobilisées, qu'en position d'utilisation, lorsqu'on déplace la coiffe 22 par rapport à l'embase 8.

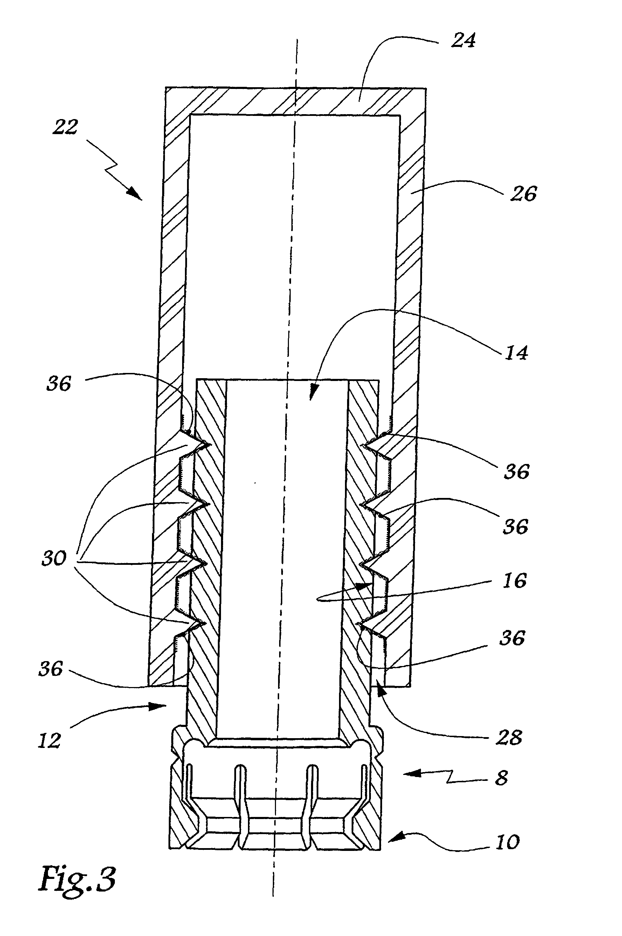

La figure 3 représente une phase de mise en place du dispositif de connexion 6 de l'invention. On insère tout d'abord, de façon connue, le piston 18 non représenté sur cette figure 3, dans l'alésage 14 de l'embase 8. Puis, on rapporte, par exemple de façon automatisée, la coiffe 22 sur la périphérie extérieure de l'embase 8. L'ensemble ainsi constitué est ensuite solidarisé au récipient 2, de manière à être stocké. Durant cette phase de stockage, les arêtes 30 pénétrent dans la paroi 16 en regard de l'embase 8, ce qui confère une étanchéité satisfaisante et évite toute migration microbienne extérieure en direction du volume intérieur de l'embase 8, et donc de l'aiguille 20. La présence du film 36 confère une composante d'étanchéité secondaire.

Immédiatement avant l'utilisation du dispositif de connexion 6, on retire la coiffe 22 de l'embase 8, par une action sensiblement verticale dirigée vers le haut. Cette action est particulièrement aisée, du fait de la présence du film 36 assurant une lubrification entre les parois en regard de la coiffe 22 et de l'embase 8. On procède alors de façon classique, à la perforation du bouchon 4 du récipient 2 au moyen de l'aiguille 20. On rapporte enfin, au voisinage de l'embase 8, le contenant auquel le récipient 2 doit être connecté.

L'invention n'est pas limitée aux exemples décrits et représentés. En effet, il peut être prévu de réaliser un nombre quelconque d'arêtes, analogues à celles 30 de la coiffe 22. Par ailleurs, il est possible de réaliser l'embase 8 en un matériau plus dur que celui constitutif de la coiffe 22. Dans ce dernier cas, la périphérie extérieure de l'embase 8 est pourvue d'arêtes, analogues à celles 30, faisant saillie radialement vers l'extérieur et coopérant avec la paroi intérieure lisse de la coiffe, la paroi lisse précitée possédant un diamètre intérieur qui est plus petit que le diamètre extérieur des arêtes de l'embase.

## Revendications

1. Procédé de fabrication d'un dispositif de connexion (6) entre un récipient fermé (2) et un contenant, ce dispositif de connexion (6) comprenant :
- une embase (8) destinée à être immobilisée sur ce récipient (2),
- un piston (18) mobile dans un alésage (14) de ladite embase (8), ce piston (18) étant pourvu d'un organe de perforation (20) apte à traverser un bouchon (4) du récipient (2), et
- une coiffe (22) recouvrant au moins partiellement la périphérie extérieure (16) de l'embase (8),
**caractérisé en ce qu'**on réalise un premier élément (22) de l'ensemble formé par l'embase (8) et la coiffe (22) en un matériau plus dur que le second élément (8) dudit ensemble (8, 22), **en ce qu'**on munit le premier élément (22) d'au moins une arête périphérique (30) destinée à pénétrer, de façon étanche, dans une région en regard du second élément (8), et **en ce qu'**on recouvre la ou chaque arête (30) du premier élément (8) au moyen d'un agent de lubrification adapté pour former, une fois cette opération de recouvrement effectuée, un film de lubrification (36) apte à rester en place sur la ou chaque arête d'étanchéité, lors du déplacement relatif de la coiffe (22) et de l'embase (8), ainsi que lorsque cette coiffe (22) et cette embase (8) sont immobiles l'une par rapport à l'autre.

2. Procédé selon la revendication 1, **caractérisé en ce que** l'agent de lubrification possède une viscosité à 20°C, comprise entre 150 et 1 500 centistokes.

3. Procédé selon l'une des revendications 1 ou 2, **caractérisé en ce que** l'agent de lubrification n'est pas miscible avec l'eau.

4. Procédé selon l'une des revendications 1 à 3, **caractérisé en ce que** l'agent de lubrification est une huile siliconée.

5. Procédé selon l'une des revendications 1 à 3, **caractérisé en ce que** l'agent de lubrification est une huile fluorée.

6. Procédé selon l'une des revendications 1 à 5, **caractérisé en ce qu'**on recouvre le premier élément (22) muni d'au moins une arête (30), par pulvérisation de l'agent de lubrification.

7. Dispositif de connexion (6) entre un récipient fermé (2) et un contenant, ce dispositif de connexion (6) comprenant :
- une embase (8) destinée à être immobilisée sur ce récipient (2),
- un piston (18) mobile dans un alésage (14) de l'embase (8), ce piston (18) étant pourvu d'un organe de perforation (20) apte à traverser un bouchon (4) du récipient (2), et
- une coiffe (22) recouvrant au moins partiellement la périphérie extérieure (16) de l'embase (8),
**caractérisé en ce qu'**un premier élément (22) de l'ensemble formé par l'embase (8) et la coiffe (22) est réalisée en un matériau plus dur que le second élément (8) dudit ensemble (8, 22), **en ce que** ledit premier élément (22) est muni d'au moins une arête périphérique (30) pénétrant, de façon étanche, dans une région en regard du second élément (8) et **en ce que** la ou chaque arête (30) du premier élément (22) est recouverte au moyen d'un film de lubrification (36) apte à rester en place sur la ou chaque arête (30), lors du déplacement relatif de la coiffe (22) et de l'embase (8), ainsi que lorsque cette coiffe (22) et cette embase (8) sont immobiles l'une par rapport à l'autre.

8. Dispositif selon la revendication 7, **caractérisé en ce que** la ou chaque arête (30) du premier élément (22) pénètre dans la paroi en regard du second élément (8), selon une profondeur comprise entre 10 et 15 micromètres.

9. Dispositif selon l'une des revendications 7 ou 8, **caractérisé en ce que** le film de lubrification (36) possède une épaisseur comprise entre 5 et 10 micromètres.

10. Dispositif selon l'une des revendications 7 à 9, **caractérisé en ce que** la différence de duretés entre les premier (22) et second (8) éléments, sur l'échelle Shore D, est comprise entre 10 et 15.

11. Ensemble prêt à l'emploi comprenant un récipient fermé (2) contenant un produit, notamment une préparation pharmaceutique, et un dispositif de connexion (6) selon l'une quelconque des revendications 7 à 10, monté sur ledit récipient (2).

## Patentansprüche

1. Verfahren zum Herstellen einer Verbindungsvorrichtung (6) zwischen einem geschlossenen Gefäß (2) und einem Behälter, wobei die Verbindungsvorrichtung (6) folgendes aufweist:
- eine Basis (8), die dazu vorgesehen ist, an dem Gefäß (2) lagefest angeordnet zu werden,
- einen Kolben (18), der in einer Bohrung (14) der Basis (8) beweglich ist, wobei der Kolben (18) mit einem Perforierungsorgan (20) versehen ist, das in der Lage ist, einen Stöpsel (4) des Gefäßes (2) zu durchstoßen, und
- eine Abdeckung (22), welche den Außenumfang (16) der Basis (8) zumindest teilweise abdeckt,
**dadurch gekennzeichnet,**
**daß** ein erstes Element (22) der durch die Basis (8) und die Abdeckung (22) gebildeten Einheit aus einem härteren Werkstoff als das zweite Element (8) der Einheit (8, 22) ausgebildet wird, dadurch, daß das erste Element (22) mit mindestens einer Umfangskante (30) versehen wird, die dazu vorgesehen ist, in dichter Weise in einen dem zweiten Element (8) gegenüberliegenden Bereich vorzudringen, sowie dadurch, daß die bzw. jede Kante (30) des ersten Elementes (8) mit einem Schmiermittel beschichtet wird, das dazu ausgelegt ist, nach der Durchführung des Beschichtungsschrittes eine Schmiermittelschicht (36) zu bilden, die in der Lage ist, auf der bzw. auf jeder Dichtkante zu verbleiben, wenn die Abdeckung (22) und die Basis (8) relativ zueinander verschoben werden, sowie wenn die Abdeckung (22) und die Basis (8) relativ zueinander festgelegt sind.

2. Verfahren nach Anspruch 1,
**dadurch gekennzeichnet,**
**daß** das Schmiermittel eine Viskosität bei 20 °C besitzt, die zwischen 150 und 1500 cSt liegt.

3. Verfahren nach einem der Ansprüche 1 oder 2,
**dadurch gekennzeichnet,**
**daß** das Schmiermittel nicht mit Wasser mischbar ist.

4. Verfahren nach einem der Ansprüche 1 bis 3,
**dadurch gekennzeichnet,**
**daß** das Schmiermittel ein silikonhaltiges Öl ist.

5. Verfahren nach einem der Ansprüche 1 bis 3,
**dadurch gekennzeichnet,**
**daß** das Schmiermittel ein fluorhaltiges Öl ist.

6. Verfahren nach einem der Ansprüche 1 bis 5,
**dadurch gekennzeichnet,**
**daß** das mit mindestens einer Kante (30) versehene erste Element (22) durch Aufsprühen mit dem Schmiermittel beschichtet wird.

7. Verbindungsvorrichtung (6) zwischen einem geschlossenen Gefäß (2) und einem Behälter, wobei die Verbindungsvorrichtung (6) folgendes aufweist:
- eine Basis (8), die dazu vorgesehen ist, an dem Gefäß (2) lagefest angeordnet zu werden,
- einen Kolben (18), der in einer Bohrung (14) der Basis (8) beweglich ist, wobei der Kolben (18) mit einem Perforierungsorgan (20) versehen ist, das in der Lage ist, einen Stöpsel (4) des Gefäßes (2) zu durchstoßen, und
- eine Abdeckung (22), welche den Außenumfang (16) der Basis (8) zumindest teilweise abdeckt,
**dadurch gekennzeichnet,**
**daß** ein erstes Element (22) der durch die Basis (8) und die Abdeckung (22) gebildeten Einheit aus einem härteren Werkstoff als das zweite Element (8) der Einheit (8, 22) ausgebildet ist, dadurch, daß das erste Element (22) mit mindestens einer Umfangskante (30) versehen ist, die in dichter Weise in einen dem zweiten Element (8) gegenüberliegenden Bereich vordringt, sowie dadurch, daß die bzw. jede Kante (30) des ersten Elementes (22) mit einer Schmiermittelschicht (36) überzogen ist, die in der Lage ist, auf der bzw. auf jeder Dichtkante zu verbleiben, wenn die Abdeckung (22) und die Basis (8) relativ zueinander verschoben werden, sowie wenn die Abdeckung (22) und die Basis (8) relativ zueinander festgelegt sind.

8. Vorrichtung nach Anspruch 7,
**dadurch gekennzeichnet,**
**daß** die bzw. jede Kante (30) des ersten Elementes (22) in die dem zweiten Element (8) gegenüberliegende Wand über eine Tiefe eindringt, die zwischen 10 und 15 µm liegt.

9. Vorrichtung nach einem der Ansprüche 7 oder 8,
**dadurch gekennzeichnet,**
**daß** die Schmiermittelschicht (36) eine Dicke besitzt, die zwischen 5 und 10 µm liegt.

10. Vorrichtung nach einem der Ansprüche 7 bis 9,
**dadurch gekennzeichnet,**
**daß** der Härteunterschied zwischen dem ersten Element (22) und dem zweiten Element (8) auf der Shore D-Skala zwischen 10 und 15 beträgt.

11. Gebrauchsfertige Einheit, die ein geschlossenes Gefäß (2) aufweist, welches ein Produkt, insbesondere ein pharmazeutisches Präparat enthält, sowie eine Verbindungsvorrichtung (6) nach einem der Ansprüche 7 bis 10, welche auf das Gefäß (2) montiert ist.

## Claims

1. Process for manufacturing a device (6) for connection between a closed recipient (2) and a container, this connection device (6) comprising:
- a base (8) intended to be immobilized on this recipient (2),
- a plunger (18) mobile in a bore (14) in said base (8), this plunger (18) being provided with a perforation member (20) adapted to traverse a stopper (4) of the recipient (2), and
- a cap (22) covering the outer periphery (16) of the base (8) at least partially,
**characterized in that** a first element (22) of the assembly formed by the base (8) and the cap (22) is made of a material which is harder than the second element (8) of said assembly (8, 22), **in that** the first element (22) is provided with at least one peripheral edge (30) intended to penetrate, tightly, in a region of the second element (8) lying opposite, and **in that** the or each edge (30) of the first element (8) is coated by means of a lubricating agent adapted, once this coating operation is effected, to form a lubricating film (36) adapted to remain in place on the or each sealing edge, during the relative displacement of the cap (22) and the base (8), and when this cap (22) and this base (8) are immobile with respect to each other.

2. Process according to Claim 1, **characterized in that** the lubricating agent has a viscosity at 20°C of between 150 and 1500 centistokes.

3. Process according to one of Claims 1 or 2, **characterized in that** the lubricating agent is not miscible with water.

4. Process according to one of Claims 1 to 3, **characterized in that** the lubricating agent is a siliconed oil.

5. Process according to one of Claims 1 to 3,
**characterized in that** the lubricating agent is a fluorinated oil.

6. Process according to one of Claims 1 to 5, **characterized in that** the first element (22) provided with at least one edge (30) is coated by spraying the lubricating agent.

7. Device (6) for connection between a closed recipient (2) and a container, this connection device (6) comprising:
- a base (8) intended to be immobilized on this recipient (2),
- a plunger (18) mobile in a bore (14) in said base (8), this plunger (18) being provided with a perforation member (20) adapted to traverse a stopper (4) of the recipient (2), and
- a cap (22) covering the outer periphery (16) of the base (8) at least partially,
**characterized in that** a first element (22) of the assembly formed by the base (8) and the cap (22) is made of a material which is harder than the second element (8) of said assembly (8, 22), **in that** the first element (22) is provided with at least one peripheral edge (30) intended to penetrate, tightly, in a region of the second element (8) lying opposite, and **in that** the or each edge (30) of the first element (8) is coated by means of a lubricating agent adapted to remain in place on the or each sealing edge, during the relative displacement of the cap (22) and the base (8), and when this cap (22) and this base (8) are immobile with respect to each other.

8. Device according to Claim 7, **characterized in that** the or each edge (30) of the first element (22) penetrates in the opposite wall of the second element (8) to a depth of between 10 and 15 micrometers.

9. Device according to one of Claims 7 or 8, **characterized in that** the lubricating film (36) has a thickness of between 5 and 10 micrometers.

10. Device according to one of Claims 7 to 9, **characterized in that** the difference in hardness between the first (22) and second (8) elements is, on the Shore D scale, between 10 and 15.

11. Ready-to-use assembly comprising a closed recipient (2) containing a product, in particular a pharmaceutical preparation, and a connection device (6) according to any one of Claims 7 to 10, mounted on said recipient (2).
